# EUROPEAN PATENT APPLICATION

(11) **EP 3 284 355 A1**
(43) Date of publication of application: **21.02.2018**
(21) Application number: 16200709.0
(22) Date of filing: 25.11.2016
(51) Int. Cl.: A24F 47/00

(54) **AN ELECTRONIC CIGARETTE TEMPERATURE CONTROL SYSTEM BASED ON A JOULE MODE**

(30) Priority: 18.08.2016 CN 201610686417
(71) Applicant: Chen, Zhenjiang, Wanning City Hainan 571525 (CN)
(72) Inventor: Chen, Zhenjiang, Wanning City Hainan 571525 (CN)
(74) Representative: Casalonga

(57) **Abstract**

The present invention provides an electronic cigarette temperature control system based on a joule mode, comprising a battery (100) for driving an electronic cigarette to operate and an atomizer for heating tobacco tar inside the electronic cigarette to atomize the tobacco tar, the atomizer being hidden inside a battery case. The atomizer is connected to a temperature controller (200) configured to control the temperature of the atomizer, and the battery (100), the temperature controller (200) and the atomizer (300) are successively and electrically connected to form an electronic cigarette temperature control system. The electronic cigarette temperature control system based on a joule mode as provided by the present invention explains the concept of the system highly accurately. The system shows controllable temperature and controllable output heat. The target temperature is controllable (by adjusting the amount of the output heat). The unit of heat is direct and accurate.

## Description

### TECHNICAL FIELD

The present invention relates to a control system for an electronic cigarette and in particular to an electronic cigarette temperature control system based on a joule mode.

### BACKGROUND OF THE PRESENT INVENTION

Electronic cigarettes, as a fashionable and technically novel product rising with the technological advancements and the improvements in the living standard, provide more flavors and tastes than those provided by general cigarettes, and relax the smokers without doing harm to human health. With regard to electronic cigarettes, the tobacco tar is usually atomized by an atomizer to be used by the users. After an attempt to go to electronic cigarettes, people's dependency on cigarettes will gradually reduce, and their desire for cigarettes will also gradually become lower.

At present, for the commercially available electronic cigarettes, they mainly operate in the following modes: mechanical mode, power mode, temperature control mode. However, they have the following disadvantages.
1. Mechanical mode: also called direct-through mode, in which a battery is connected to an atomizer so that the atomizer generates heat which heats the tobacco tar to produce smoke. The mechanical mode has the disadvantages that the power is unitary and non-adjustable; and there is no protection function, and correspondingly, when in use, the atomizer may be short-circuited and there may be a risk of explosion due to the short-circuit of the battery. The mechanical mode shows low safety.
2. Power mode: the amount of smoke of electronic cigarettes is adjusted by changing the power simply by controlling the output power. (The function is unitary and the experience is poor.) Furthermore, because of the constant power, when the atomizer continues to operate when its power reaches a certain degree, excessive power consumption of the battery and waste of tobacco tar due to its running-dry will be caused since the power is too high. Burning out the oil-immersed cotton of the atomizer due to a too high power will degrade the experience of the electronic cigarette (undesirable flavor, smells burning).
3. Temperature control: this mode is mainly embodied in control by temperature. The unit usually used in this mode is degree Celsius or degree Fahrenheit, and the concept of the relation between the output power and the temperature is fuzzy. Usually, it is set to be controlled by the temperature, but the power at that point of time is not directly and clearly indicated. Or, the temperature control mode is conceptually confused with the non-temperature control mode (power mode). As a result, when in use, it is difficult for the users to distinguish whether it is a power mode or a temperature control mode.

Generally, the temperature control mode in the commercially available electronic cigarettes purely means temperature setting.

### SUMMARY OF THE PRESENT INVENTION

To solve the deficiencies in the prior art, an objective of the present invention is to provide an electronic cigarette temperature control system based on a joule mode.

To achieve this objective of the present invention, the following technical solutions are employed.

The present invention provides an electronic cigarette temperature control system based on a joule mode, comprising a battery for driving an electronic cigarette to operate and an atomizer for heating tobacco tar inside the electronic cigarette to atomize the tobacco tar, the atomizer being hidden inside a battery case, characterized in that the atomizer is connected to a temperature controller configured to control the temperature of the atomizer, and the battery, the temperature controller and the atomizer are successively and electrically connected to form an electronic cigarette temperature control system; and
the temperature controller controls different atomizer resistance values to operate in a set joule mode and is cut off when a resistance value of the atomizer exceeds a set joule value, and the battery stops supplying power to the atomizer.

Further, the temperature controller detects a voltage required to obtain a set amount of heat under different atomizer resistance values, and controls a voltage output by the atomizer; and in the event of generating heat by the atomizer, the change in a temperature curve for resistance values is automatically matched with an output voltage.

Further, the temperature control system is built on the basis of an electronic system, and further comprises a battery over-charge/over-discharge protection module, an output low-resistance protection module and a battery reverse connection module, which are all connected to the temperature controller; and both the battery over-charge/over-discharge protection module and the battery reverse connection module are connected to the battery, and the output low-resistance protection module is connected to the atomizer.

Further, the electronic system is a PCB.

Further, the battery over-charge/over-discharge protection module is configured to protect the battery when the battery is charged to a value greater than a set value or discharged to a value lower than a set value; the output low-resistance protection module is configured to protect the atomizer when the output of the atomizer is in low resistance; and the battery reverse connection module is configured to protect the battery when the battery cannot be charged.

Further, the joule is defined in the field of heat energy as follows: 1 joule =1 Watt • second; and the unit of the joule is J.

Further, the temperature control system is a single-chip microcomputer controller, an MCU controller or a PLC controller.

Further, the temperature control system further comprises a liquid crystal display screen which is connected to the temperature controller.

A simplified summary is provided herein to help enable a basic understanding of some aspects of the disclosed embodiments. This summary is not intended, however, as an extensive overview nor to identify key/critical elements or to delineate the scope of these embodiments. Its sole purpose is to present some concepts in a simplified form as a prelude to the detailed description that follows.

Compared with the closest prior art, the technical solutions of the present invention have the following beneficial effects.

The present invention is mainly based on the temperature control concept for an electronic cigarette control system. Other concepts are merely embodied in temperature. And, during the course from a low temperature to a high temperature, the unit of heat used is absent or represented by the unit of power (watt). When in use, to reach the set temperature value, it is necessary to increase the output heat. However, such control is not provided for some commercially available electronic cigarettes. The electronic cigarette temperature control system based on a joule mode as provided by the present invention explains the concept of the system highly accurately. The system shows controllable temperature and controllable output heat. The target temperature is controllable (by adjusting the amount of the output heat). The unit of heat is direct and accurate.

For the foregoing and related purposes, one or more embodiments include the features that will be illustrated in detail below and specifically recited in the claims. The following description and drawings illustrate some exemplary aspects in detail; moreover, it only indicates some of the various modes in which the principle of each embodiment may be applied. Other benefits and novel features will be apparent from the following detailed illustration in conjunction with the drawings, and all the embodiments disclosed intend to contemplate all these aspects and their equivalents.

### BRIEF DESCRIPTION OF THE DRAWINGS

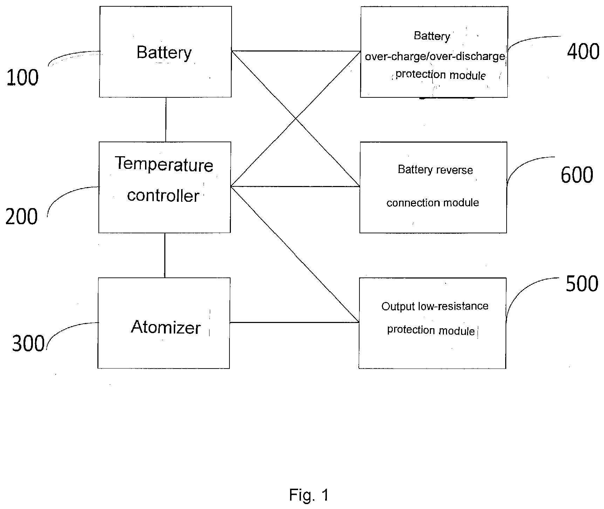

Fig. 1 is a schematic diagram of an electronic cigarette temperature control system based on a joule mode according to the present invention, in which:
100: battery;
200: temperature controller;
300: atomizer;
400: battery over-charge/over-discharge protection module;
500: output low-resistance protection module; and
600: battery reverse connection module.

### DETAILED DESCRIPTION OF THE PRESENT INVENTION

Hereinafter, the specific implementations of the present invention will be further described in detail with reference to the accompanying drawings.

The following description and the accompanying drawings fully illustrate specific embodiments of the present invention, to enable one skilled in the art to implement the embodiments. Modifications, such as structural, logical, electrical and process modifications, can be made in other embodiments. The embodiments represent some possible variations. Individual components or functions are optional and the operation order is variable, unless otherwise stated specifically. A part and certain feature of some embodiments may be included in or replaced by a part and certain feature of other embodiment. The scope of the embodiments of the invention includes the whole scope of the claims and all obtainable equivalents thereof. Herein, these embodiments of the present invention may be individually or generally represented by the term "invention" for the sake of convenience; moreover, if more than one invention is disclosed actually, it is not intended automatically to limit the application scope to any individual invention or inventive concept.

### Embodiment

Adhering to the principles of safety, high efficiency and user-friendly, improvements and replacements have been done to overcome the disadvantages described above. The present invention provides an electronic cigarette temperature control system based on a joule mode, comprising a battery 100 for driving an electronic cigarette to operate and an atomizer 300 for heating tobacco tar inside the electronic cigarette to atomize the tobacco tar, the atomizer 300 being hidden inside a battery case. The atomizer 300 is connected to a temperature controller 200 configured to control the temperature of the atomizer, and the battery 100, the temperature controller 200 and the atomizer 300 are successively and electrically connected to form an electronic cigarette temperature control system.

The temperature controller 200 controls different atomizer 300 resistance values to operate in a set joule mode and is cut off when a resistance value of the atomizer 300 exceeds a set joule value, and the battery 100 stops supplying power to the atomizer 300. The temperature controller 200 detects a voltage required to obtain a set amount of heat under different atomizer resistance values, and controls a voltage output by the atomizer; and in the event of generating heat by the atomizer, the change in a temperature curve for resistance values is automatically matched with an output voltage.

By combining the change curve of the resistance value and the change curve of the temperature in the event of generating heat by the atomizer, the electronic cigarette is allowed to perform accurate heat and temperature output control.

The temperature control system is built on the basis of an electronic system, and further comprises a battery over-charge/over-discharge protection module 400, an output low-resistance protection module 500 and a battery reverse connection module 600, which are all connected to the temperature controller; and the output low-resistance protection module 500 is connected to the atomizer 300. The electronic system is a PCB. The battery 100 over-charge/over-discharge protection module is configured to protect the battery when the battery is charged to a value greater than a set value or discharged to a value lower than a set value; the output low-resistance protection module 500 is configured to protect the atomizer 300 when the output of the atomizer 300 is in low resistance; and the battery reverse connection module 600 is configured to protect the battery 100 when the battery cannot be charged.

The joule is defined in the field of heat energy as follows: 1 joule =1 Watt • second; and the unit of the joule is J. The temperature control system is a single-chip microcomputer controller, an MCU controller or a PLC controller. The temperature control system further comprises a liquid crystal display screen which is connected to the temperature controller 200.
1. The temperature control system is intended to build an electronic cigarette temperature control system containing a main power mode and a joule mode on the basis of an electronic system covering various protection measures such as battery over-charge/over-discharge protection, output low-resistance protection, output protection and battery reverse connection.
2. The present invention defines a novel unit for heat for electronic cigarettes: joule (unit: J), which is defined in the field of heat energy as follows: 1 joule (J) =1 Watt • second (W • s). This fully distinguishes the operation of the electronic cigarette system in the temperature control mode and in the power mode. Thus, the recognition of the temperature control mode becomes more accurate.
3. The operating principle of the electronic cigarette temperature control system based on a joule (J) mode is a process in which the output joule (heat generated per second) and the temperature are set by the electronic cigarette control system in such a way that a single-chip microcomputer detects a voltage required to obtain a set amount of heat under different atomizer resistance values and controls a voltage output by the atomizer, and in the event of generating heat by the atomizer, the change in a curve for resistance values is automatically matched with a proper output voltage. By combining the change curve of the resistance value and the change curve of the temperature, the electronic cigarette is allowed to perform accurate heat and temperature output control. Therefore, the temperature control system based on a joule mode has no disadvantages of unitary power, over-heating and burning as those occurred in the conventional electronic cigarettes under the power mode. Accordingly, excellent experience, such as good taste, high efficiency and user-friendly, is promised.

In the above detailed description, various features are combined in a single embodiment for the sake of simplification of the disclosure. This disclosing manner should not be interpreted as reflecting such an intention that: the embodiment of the claimed subject requires more features than those stated clearly in each claim. On the contrary, as reflected in the appended claims, the invention may be in a state with fewer features than all features of a single disclosed embodiment. Therefore, the appended claims are hereby incorporated in the detailed description clearly, and each claim independently presents an individual preferred implementation solution of the invention.

Finally, it is to be noted that the above embodiments are provided for describing but not for limiting the technical solutions of the present invention. Although the present invention has been described in detail with reference to the above embodiments, modifications or equivalent replacements still can be done by a person of ordinary skill in the art to the specific implementations of the present invention, and those modifications and equivalent replacements without departing from the spirit and scope of the present invention shall be regarded as falling into the protection scope of the claims of the present invention to be granted.

## Claims

1. An electronic cigarette temperature control system based on a joule mode, comprising a battery for driving an electronic cigarette to operate and an atomizer for heating tobacco tar inside the electronic cigarette to atomize the tobacco tar, the atomizer being hidden inside a battery case, **characterized in that** the atomizer is connected to a temperature controller configured to control the temperature of the atomizer, and the battery, the temperature controller and the atomizer are successively and electrically connected to form an electronic cigarette temperature control system; and
the temperature controller controls different atomizer resistance values to operate in a set joule mode and is cut off when a resistance value of the atomizer exceeds a set joule value, and the battery stops supplying power to the atomizer.

2. The electronic cigarette temperature control system according to claim 1, **characterized in that** the temperature controller detects a voltage required to obtain a set amount of heat under different atomizer resistance values, and controls a voltage output by the atomizer; and in the event of generating heat by the atomizer, the change in a temperature curve for resistance values is automatically matched with an output voltage.

3. The electronic cigarette temperature control system according to claim 1, **characterized in that** the temperature control system is built on the basis of an electronic system, and further comprises a battery over-charge/over-discharge protection module, an output low-resistance protection module and a battery reverse connection module, which are all connected to the temperature controller; and both the battery over-charge/over-discharge protection module and the battery reverse connection module are connected to the battery, and the output low-resistance protection module is connected to the atomizer.

4. The electronic cigarette temperature control system according to claim 3, **characterized in that** the electronic system is a PCB.

5. The electronic cigarette temperature control system according to claim 3, **characterized in that** the battery over-charge/over-discharge protection module is configured to protect the battery when the battery is charged to a value greater than a set value or discharged to a value lower than a set value; the output low-resistance protection module is configured to protect the atomizer when the output of the atomizer is in low resistance; and the battery reverse connection module is configured to protect the battery when the battery cannot be charged.

6. The electronic cigarette temperature control system according to claim 1, **characterized in that** the joule is defined in the field of heat energy as follows: 1 joule =1 Watt • second; and the unit of the joule is J.

7. The electronic cigarette temperature control system according to claim 1, **characterized in that** the temperature control system is a single-chip microcomputer controller, an MCU controller or a PLC controller.

8. The electronic cigarette temperature control system according to claim 1, **characterized in that** the temperature control system further comprises a liquid crystal display screen which is connected to the temperature controller.
